(19) Europäisches Patentamt
European Patent Office
Office européen des brevets

(11) **EP 2 389 885 A1**

(12) **EUROPEAN PATENT APPLICATION**

(43) Date of publication:
**30.11.2011 Bulletin 2011/48**

(51) Int Cl.:
*A61B 18/04* (2006.01) *A61F 7/00* (2006.01)

(21) Application number: **11174303.5**

(22) Date of filing: **17.05.2006**

(84) Designated Contracting States:
**AT BE BG CH CY CZ DE DK EE ES FI FR GB GR HU IE IS IT LI LT LU LV MC NL PL PT RO SE SI SK TR**

(30) Priority: **18.05.2005 US 682229 P**

(62) Document number(s) of the earlier application(s) in accordance with Art. 76 EPC:
**06760108.8 / 1 885 252**

(71) Applicant: **Zeno Corporation**
**Houston, TX 77067 (US)**

(72) Inventors:
  • **Conrad, Robert**
   **Spring, TX Texas 77379 (US)**
  • **Conrad, Charles**
   **77379, TX Texas Spring (US)**
  • **Klemp, Walter V.**
   **Houston, TX Texas 77019 (US)**

  • **Wucher, James**
   **Allen, TX Texas 75002 (US)**
  • **Dumas, Robert**
   **McKinney, TX Texas 75070 (US)**
  • **Rogers, Jeff**
   **Plano, TX Texas 75025 (US)**
  • **Martin, Robert Geoffrey**
   **Seattle, WA Washington 98103 (US)**
  • **Lord, Randall Dean**
   **Mukilteo, WA Washington 98275 (US)**

(74) Representative: **Jackson, Richard Eric**
**Carpmaels & Ransford**
**One Southampton Row**
**London**
**WC1B 5HA (GB)**

Remarks:
This application was filed on 18-07-2011 as a divisional application to the application mentioned under INID code 62.

(54) **Treatment device and method for treating skin lesions through application of heat**

(57)    The present invention relates to methods and devices for treatment of skin diseases, lesions and irritations. More specifically, the present invention relates to methods and devices for the treatment of skin lesions involving the application of a controlled dose of thermal energy to the infected or irritated tissue.

*FIG. 1*

EP 2 389 885 A1

**Description**

BACKGROUND OF THE INVENTION

Skin infections and irritations pose significant health and cosmetic problems. Bacterial and fungal skin infections lead to common lesions such as acne, pimples and under-nail fungal infections. Other lesions are caused by irritants, which may be introduced as a result of bug bites or by exposure to other natural or man-made skin irritants. Still other skin lesions are caused by viral infection, a common example being the lesions known as "cold sores" or "fever blisters". These skin lesions are often unsightly and painful, and current methods of treatment are often inadequate.

Pustular eruptions, localized abscessed formations and local inflammatory conditions of the dermis and epidermis represent a particularly significant cosmetic and health problem. One of the most common afflictions of this type are lesion caused by the condition known as acne vulgaris. Acne vulgaris is associated with the Gram-positive anaerobic bacterium, Propionibacterium acnes. Acne afflicts 90% of all teenagers, and often continues to afflict men and women in the second, third and forth decade of life, sometimes persisting throughout adulthood. (Yonkosky, D.M, and P.E. Pochi, Acne vulgaris in childhood. Pathogenesis and management. Dermatol Clin, 1.986. 4(1): p. 127-36.) Abscess formation from a number of primarily bacterial species (commonly Staphylococcus and Streptococcus) as well as fungal species, such as dermatophytes, are a less frequent medical and cosmetic problem but share similar challenges regarding effective treatment.

Setting the scene of acne and other skin infections, endogenous hormones (mainly androgens), which are present in unusually high concentrations in the blood during adolescence and puberty, give rise to an excessive production of sebum. This condition may worsen by a simultaneous increase in the rate of keratinization of the skin's horny layer (the stratum corneum). As the horny cells proliferate, they can form an occlusive plug or comedone which, coupled with the increased production of the sebum, represents an ideal medium for the proliferation of bacterial strains frequently resident on skin, such as P. acnes.

In acne vulgaris, plugged follicles eventually rupture, allowing discharge of their contents and causing local swelling and inflammation. The exposed follicles may darken from the deposition of pigment from damaged cells in the deeper layer of skin.

Acne vulgaris is therefore a chronic disorder of the pilosebaceous follicles characterized by comedones (blackheads), papules, pustules, cysts, nodules, and often results in the formation of permanent scars (Cunliffe, W.J., et al., Comedogenesis: some aetiological, clinical and therapeutic strategies. Dermatology, 2003. 206(1):11-6) that appear on the most visible areas of the skin particularly on the face, chest, back and occasionally neck, and upper arms. It is known that P. acnes also produces low-molecular-weight chemotactic factors which attract leulcocytes, thereby causing or enhancing inflammation (Scholdgen, W., Hautarzt, 1965. 16(11):518-20; Lever, L. and R. Marks, Drugs, 1990. 3 9(5): 681-92). This increased inflammatory process, if left untreated, can produce significant immediate and long-term cosmetic problems including permanent scar formation.

Acne is a multistage condition. In its most severe form it leads to hospitalization of the patient, extensive discomfort and long term scarring of the skin.

Multiple treatment options have been available for acne and localized abscess formations (Scholdgen, W., Hautarzt, 1965. 16(11):518-20; Lever, L. and R. Marks, Drugs, 1990. 39(5):681-92) since the early 1960's, however no one drug appears effective against all distinctive types of acne or abscess formation and most preparations have significant side effects. (Russell, J.J., Am Fam Physician, 2000. 61(2):357-66.) Comedolytic agents, for example, promote comedonal drainage but also cause significant skin irritation. Topical antibiotics decrease the number of mild to moderate inflammatory lesions by inhibiting the growth of P. acnes and are also associated with skin irritation, dryness, and potential antibiotic resistance as well as potential overgrowth of fungal or yeast infections. (Gollnick, H.P. and A. Krautheim, Dermatology, 2003. 206(1):29-36.) Oral antibiotics are the standard for treating moderate to severe acne lesions, however, superinfection may occur with long-term exposure and may require routine laboratory monitoring. Antibiotic treatment against P. acnes has been the mainstay of treatment for more than 40 years. (Loveckova, Y. and I. Havlikova, Biomed Pap Med Fac Univ Palacky Olomouc Czech Repub, 2002. 146(2):29-32.) Despite the widespread use of systemic antibiotics such as tetracyclines, erythromycins (Vermeulen, B., J.P. Remon, and H. Nelis, Int J Pharm, 1999. 178(1): 137-41) and clindamycins (Rizer, R.L., et al., Clindamycin phosphate 1% gel in acne vulgaris. Adv Ther, 2001. 18(6): 244-52) as the most common, changes in the sensitivity of P. acnes to antibiotics has been seen for the last two decades. A number of mutations have been characterized which lead to increased resistance of P. acnes to both systemic and topical antibiotic treatments.

Another widespread treatment option for P. acnes has been the use of oral Vitamin A acid derivatives such as cis-Retinioc Acid (Accutane). However, the use of cis-Retinoic Acid has been reserved for severe cases of acne vulgaris since significant side effects can be seen with the use of cis-Retinioc acid. (Thorne, E.G., Br J Dermatol, 1992. 127 Suppl 41:31-6.) Some of these side effects include liver toxicity, severe skin drying, increase sensitivity to UV radiation, elevations in triglicyride and cholesterol levels, as well as mood changes including severe depression. Again, cis-Retinoic Acid has been reserved for severe or refractory cases of acne vulgaris.

In addition to prescription medications for the treatment of acne vulgaris, a number of over the counter topical preparations are widely used as well. (Scholdgen, W., Z Allgemeinmed, 1972. 48(17):833-5; Melski, J.W. and K.A. Arndt, Current concepts: topical therapy for acne. N Engl J Med, 1980. 302(9):503-6; Lester, R.S., Topical formulary for the pediatrician. Pediatr Clin North Am, 1983. 30(4):749-65; Broniarczyk-Dyla, G. and C. Arkuszewska, Dermatol Monatsschr, 1989. 175(1):40-3; Zander, E. and S. Weisman, Treatment of acne vulgaris with salicylic acid pads. Clin Ther, 1992. 14(2):247-53; Kaye, E.T. and K.M. Kaye, Topical antibacterial agents. Infect Dis Clin North Am, 1995. 9(3):547-59.)

These include, broadly, drying agents, oxidizing agents and astringents, also a wide variety of skin detergents and cleansers, as well as preparations, which attempt to form oxidizing agents which are reportedly toxic to *P. acnes.*

Other treatment methods that have been suggested include the methods disclosed in U.S. Pat. No. 6,183,500 involving the use of phototherapy in the treatment of acne vulgaris, whereby a concentrated light source is used as a treatment. Additionally, ultrasound devices to deliver energy in a localized fashion have also been decribed. (Ruiz-Esparza, J. and J.B. Gomez, Dermatol Surg, 2003. 29(4):333-9; discussion 339.) Even using cautery with local anesthesia has been described. (Pepall, L.M., M.P. Cosgrove, and W.J. Cunliffe, Br J Dermatol, 1991. 125(3):256-9.) Many of these devices require expensive and unwieldy equipment, and treatment by a physician.

Other types of bacterial skin lesions include bacterial folliculitis, (a localized infection of hair follicles) dermatitis, cellulitis, impetigo, ecthyma, furuncles and the like.

It has long been known that the application of heat to both pustular eruptions as well as localized abscesses can be an effective way to treat these conditions. The most common method employed uses hot compresses, which generally must be applied multiple times throughout the day to be even marginally effective. Often the use of hot compresses is recommended to alleviate discomfort by "popping" pimples and other pustular eruptions and allowing them to drain. Although it is well-known that the application of heat is toxic to multiple forms of bacteria, including *P. acnes* and *Staphylococus* species, the use of hot compresses has shown limited utility in the treatment of skin lesions such as acne. In fact, many clinicians disfavor hot compresses because they are believed to aggravate acne. Furthemore, hot compresses are generally non-uniform in the amount of heat delivered. Over-heating of the compresses by the user may easily result in burns. Other disadvantages include the fact that hot compresses generally only maintain heat for a very limited period of time, and when moved about or reused may result in spread of infectious agents to healthy tissue.

A further type of skin lesion that has proved difficult to treat are viral skin lesions such as cold sores, also known as fever blisters. Cold sores are usually caused by strains of the Herpes Simplex virus and commonly result in lesions on and near the lips and inside the mouth of an infected individual. The sores are painful and unsightly, and like other facial lesions, frequently result in psychological stresses for the patients suffering from the condition. The eruption of the sores is often, but not always, preceded by a painful sensation that warns of an impending lesion.

Various ointments and skin treatments exist that may be used to reduce the painful symptoms of the sores and to decrease the time for the sores to heal. Certain anti-viral medications, such as Acyclovir and Famvir, may also be used to prevent outbreaks and reduce healing time. However these medications are generally expensive and only available with a prescription. Furthermore, they may result in adverse side effects such as renal toxicity and therefore physicians are sometimes reluctant to prescribe these medications for simple outbreak cases. Also, to effectively prevent a cold sore outbreak, the medications usually must be taken prophylactically or upon the first sign of an outbreak. Once the sore has erupted, the lesions generate infectious particles which may in turn infect other individuals. Alkali inhibition is commonly used for laboratory inhibition of Herpes viruses, but application of alkali is impractical in a clinical setting due to the harshness of the treatment to normal skin.

A further type of skin lesions are fungal infections, also known as fungal dermatitis, including conditions known medically as Tinea corporis, Tinea pedis, Tinea unguium, Tinea capitis, Tinea cruris, and Tinea barbae. Particularly troublesome is the condition known as Tinea unguium which is a fungal infection occuring under toenails or fingernails, a condition also referred to medically as onychomycosis or ringworm of the nails. Onychomycosis may be caused by several types of fungi, including Trichophyton mentagrophytes, Candida albicans or Trichophyton rubrum. Such infections are extremely difficult to treat effectively due to the difficulty in delivering effective amounts of antifungal medications to the area beneath the nail.

Onychomycosis can cause the nail to appear thickened and lusterless, and often causes nail discomfort. Also, the infected nail harbors a reservoir of pathogenic organisms which can spread to and re-infect other parts of the body, causing chronic diseases such as onychomycosis in other nails, athletes foot, foot dry skin and the like. Onychomycosis is prevalent throughout a large proportion of the population, with most of those afflicted from the ages of 40 years and older.

A human's nail has a nail plate, which is a hard outer surface of dead cells, and a nail bed below the nail plate. The nail plate is non-porous, whereas the nail bed is porous. There is soft flesh beneath the nail bed. The nail plate and the nail bed are relatively insensitive to pain. The underlying flesh is sensitive to pain. In onychomycosis, the nail plate, nail bed, and, in severe cases, the flesh below the nail bed can be infected.

Methods of treating onychomycosis include various methods of delivering medication to the nail bed, including various methods of introducing medication under or through the nail plate or of removing the nail plate partially or entirely to access the infected tissue. Other treatments include systemic anti-fungal medications. The difficulty with systemic medications is that they are not localized to the nail area and therefore it is difficult to achieve an effective dose without producing undesirable side effects in other parts of the body.

Tinea corporis, also known as tinea circinata or tinea glabrosa and referred to generally as ringworm of the body, is a fungal infection or dermatophytosis of the glabrous skin, i.e., areas of skin other than bearded area, scalp, groin, hands and feet, generally caused by fungal species such as those of *Microsporum* such as *Microsporum canis*, *Trichophyton* such as *Trichophyton rubrum*, *T. Mentagrophytes*, and *Epidermophyton*, particularly by the fungal species of *Trichophyton* and *Epidermophyton*. The condition generally includes the presence of one or more well-demarcated erythematous, scaly mascules with slightly raised borders and central healing, producing annular outlines. Various other types of lesions may also occur, such as those that are vesicular, eczematous, psoriasiform, verrucous, plaque-like, or deep.

Tinea cruris, also referred to generally as "jock itch" or ringworm of the groin, is a fungal infection or dermatophytosis of the groin, perineum and perineal regions, generally seen in males, and sometimes spreading to contiguous areas, generally caused by fungal species such as those of *Microsporum*, *Trichophyton*, and *Epidermophyton*, particularly by the fungal species of *Trichophyton* and *Epidermophyton.* The condition generally includes severely pruritic, sharply demarcated lesions with a raised erythematous margin and thin, dry scaling. Tinea cruris often accompanies tinea pedis (also known as "athlete's foot").

Tinea pedis results in interdigital lesions. Athlete's foot is an itching, malodorous, uncomfortable disorder resulting from large numbers of ordinary, nonvirulent bacteria proliferating in the fungus infected interspace.

Certain insect bites and contact with certain plants can expose skin to irritants that result in an itchy or painful immune response. The symptoms generally manifest soon after the introduction of the irritant, but can persist or sporadically reoccur for extended periods of time when the irritant is not effectively removed or inactivated by the immune response. Various treatments have been proposed for the treatment of the symptoms caused by these irritants. Typically the treatment involves that application of compounds that inhibit the immune response that generates the itching and inflammation usually associated with these conditions. These compounds tend to mask the symptoms of the insect bite without addressing the root cause of the irritation. They also tend to require repeated applications in order to obtain continuous symptom relief and frequently do not speed healing time in any appreciable manner.

For insect bites, a device has recently been marketed that is known as an "ItchZapper™". This device allegedly treats insect bites by applying one or more bursts of heat to the area of the bite thereby breaking down the irritants introduced by the insect bite and stopping the release of histamine. The device represented as heating to a temperature of 122°F, and insect proteins are said to break down at 118°F. The ItchZapper™ device heats to a peak temperature over a period of 2 to 4 seconds. The device cools as residual heat bled off the device for a few seconds after the heating cycle was completed. The upward and downward ramping of the temperature is pronounced and the device is not capable of holding a sustained temperature for any appreciable period of time. Additionally, the extremely brief treatment period is unlikely to have any appreciable effect on insect bite symptoms without repeated treatments.

There is therefore a need for improved treatments for skin lesions caused by bacterial, viral and fungal infections and by exposure to irritants such as those introduced by insect bites and poisonous plants, particularly treatments that will effectively ameliorate the symptoms of the lesions and promote healing without causing adverse effects in the majority of patients.

BRIEF SUMMARY OF THE INVENTION

This invention relates to the use of a regulated heat source that can be applied to a skin lesion, such as pustular-form eruption or localized abscess, in order to accelerate the death of invading bacteria, fungi or viral particles, or to assist in the breakdown of a skin irritant and thereby speed the recovery process.

The foregoing has outlined rather broadly the features and technical advantages of the present invention in order that the detailed description of the invention that follows may be better understood. Additional features and advantages of the invention will be described hereinafter which form the subject of the claims of the invention. It should be appreciated by those skilled in the art that the conception and specific embodiment disclosed may be readily utilized as a basis for modifying or designing other structures for carrying out the same purposes of the present invention. It should also be realized by those skilled in the art that such equivalent constructions do not depart from the spirit and scope of the invention as set forth in the appended claims. The novel features which are believed to be characteristic of the invention, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present invention.

The foregoing has outlined rather broadly the features and technical advantages of the present invention in order that the detailed description of the invention that follows may be better understood. Additional features and advantages of the invention will be described hereinafter which form the subject of the claims of the invention. It should be appreciated by those skilled in the art that the conception and specific embodiment disclosed may be readily utilized as a basis for modifying or designing other structures for carrying out the same purposes of the present invention. It should also be realized by those skilled in the art that such equivalent constructions do not depart from the spirit and scope of the invention as set forth in the appended claims. The novel features which are believed to be characteristic of the invention, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present invention.

BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

FIGURE 1 shows a perspective view of an embodiment of treatment device according to the present invention;

FIGURE 2a shows a side view of an embodiment of a replaceable treatment tip according to the present invention;

FIGURE 2b shows a perspective view of the replaceable treatment tip of FIGURE 2a;

FIGURE 3 shows a simplified block diagram of the major electrical components treatment device of FIGURE 1;

FIGURE 4 is a diagram illustrating the control functionality of the firmware used in the present invention;

FIGURE 5 shows a state diagram illustrating the operation of a treatment device according to an embodiment of the present invention;

FIGURE 6 is a perspective view of an embodiment of a treatment device capable of treating multiple locations simultaneously according to the present invention;

FIGURE 7 is a perspective view of an embodiment of a treatment device capable of wireless treatment of multiple locations simultaneously according to the present invention;

FIGURE 8 is a perspective view of a treatment tip for use with the treatment device of FIGURE 5 or FIGURE 6; and

FIGURE 9 shows a graph of the Thermal Aspect Ratio in accordance with an embodiment of the present invention.

FIGURE 10 shows temperature death curves for P. acnes.

DETAILED DESCRIPTION OF THE INVENTION

The present invention describes methods and devices for the treatment of skin diseases, lesions, irritants, and other localized skin conditions, collectively "lesions", involving the application of a controlled dose of thermal energy to the infected or irritated tissue and thereby speeding the recovery process. A lesion according to the present invention is any infected or irritated tissue caused by bacterial, fungal or viral infections, or other type of skin irritant, which can be treated through the application of a regulated amount of heat. The invention can also be used to cause the thermal breakdown of certain skin irritants. For the purposes of the present invention "treating" a skin lesion means to slow, halt or even reverse the development of skin lesions and to reduce the lesion's healing time.

Lesions To Be Treated

Skin lesions of the dermis, epidermis, follicle or other cutaneous structures can be treated by the methods and devices of the present invention, as well as skin lesions on mucosal surfaces such as the gums or other skin on the inside of the mouth. Additional skin structures in and around the finger or toe nails and cuticle are also potential sites prone to develop bacterial and fungal infections.

The lesions can be the result of infection by a bacterial strain including but not limited to strains such as Propionibacterium acnes, Staphylococcus species or Streptococcus species. In preferred embodiments, the present invention provides methods and devices for the treatment of skin lesions such as the kind commonly associated with acne vulgaris. These skin lesions include pustular eruptions and localized abscesses such as cysts, nodules, pustules, papules, comedones (blackheads) and the like. These lesions include those that are commonly referred to as pimples, whiteheads, zits, acne and the like.

Alternatively or additionally, the lesions can further be result of infection by fungal species, including but not limited to fungal species capable of producing conditions such as toenail or fingernail infections, ringworm and the like. These fungal species include Microsporum species such as Microsporum canis, Trichophyton species such as Trichophyton rubrum, Trichophyton. Mentagrophytes, Epidermophyton species, Candida albicans, and the like. Such fungal species are sometimes referred to broadly as "dertnatophytes".

Alternatively or additionally, in other embodiments, the skin lesions can be the result of viral infections, including infections caused by Herpes viruses such as Herpes simplex types I and II (cold sores and genital herpes), Varicella zoster (chicken pox) and the like.

While not bound by theory, it is believed that treatment of skin lesions caused by bacterial, fungal and viral infections can be effectively treated by the application of controlled quantities of heat either by the stimulation of a "heat-shock" response in the microorganism resulting in its death, impairment, dormancy or other loss of viability of the infectious agent.

Alternatively or additionally, embodiments of the present invention provide methods and devices for the controlled application of heat for the treatment of skin lesions caused by an irritant. Common skin irritants that can be treated by the present invention include those introduced by bug bites, such as mosquito, chigger, ant, spider bites, scabies and the like. Other skin irritants introduced by other animal species, such as jellyfish, stingrays, snakes and the like, or by plants such as poison ivy, poison oak, poison sumac and the like, can also be treated using the methods and devices of the present invention. Not be limited by theory, the application of regulated quantities of heat can result in the biochemical denaturation of the foreign irritant proteins, or can disrupt the host reaction to the particular irritant, or both. The disruption of the host reaction can occur by the heat producing an affect on the cellular response to the foreign material.

The methods and devices of the present invention provide the application to a lesion of an amount of heat (thermal energy) wherein the heat is applied over one or more treatment periods in an amount sufficient to result in improved recovery times for the treated lesion. An effective therapeutic amount is therefore any application or applications of heat that are capable of measurably decreasing average recovery times for a given type of skin lesion, preferably by improving the average recovery time by 1, 2, 3, 4, 5 or more days, preventing nascent outbreaks of new lesions, and additionally or alternatively, appreciably or substantially reducing the discomfort associated with the lesion, such as itching or sensations of pain, pressure, heat and the like.

Devices and methods of treating skin lesions

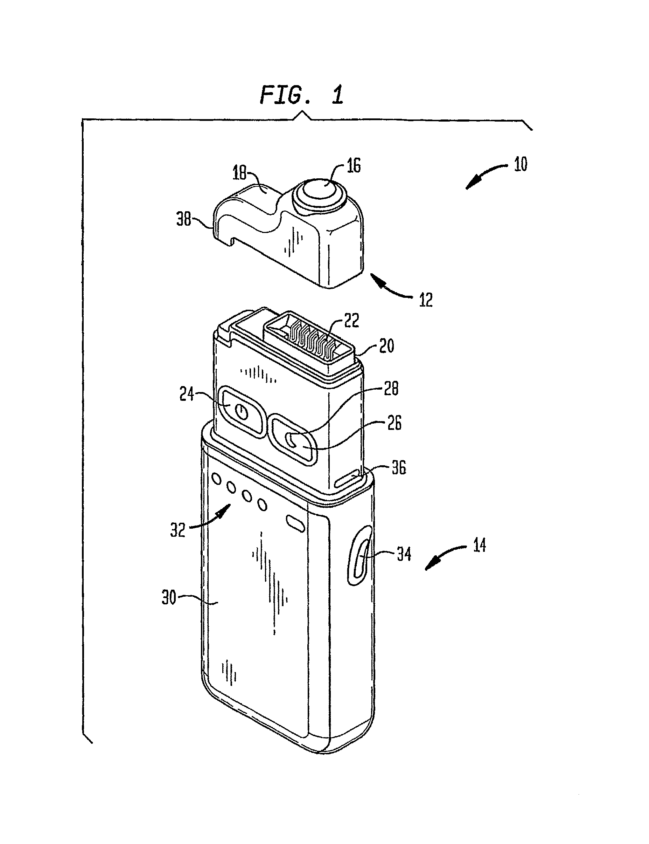

Referring now to Figure 1, an embodiment of a device for treating individual skin lesions is described. Treatment device 10 operates to transfer heat energy to a skin lesion at a set temperature for a set period of time. The set temperature and set period of time can be varied to accommodate different skin lesions, but embodiments of treatment device 10 should be capable of heating a treatment surface to a temperature between 38°C and 67°C and sustaining one or more temperatures within that range for at least 5 seconds, but, in most cases, for between 60 seconds and 240 seconds. Although thermal damage generally occurs when human skin is heated to a temperature of approximately 66°C (150°F) or greater, an interface according to an embodiment of the invention heated to this temperature or a higher temperature can nevertheless deliver an effective therapeutic amount of heat to a lesion without resulting in thermal damage, depending on the amount of thermal energy delivered over a particular surface area and how readily the thermal energy is dissipated by the heated tissue.

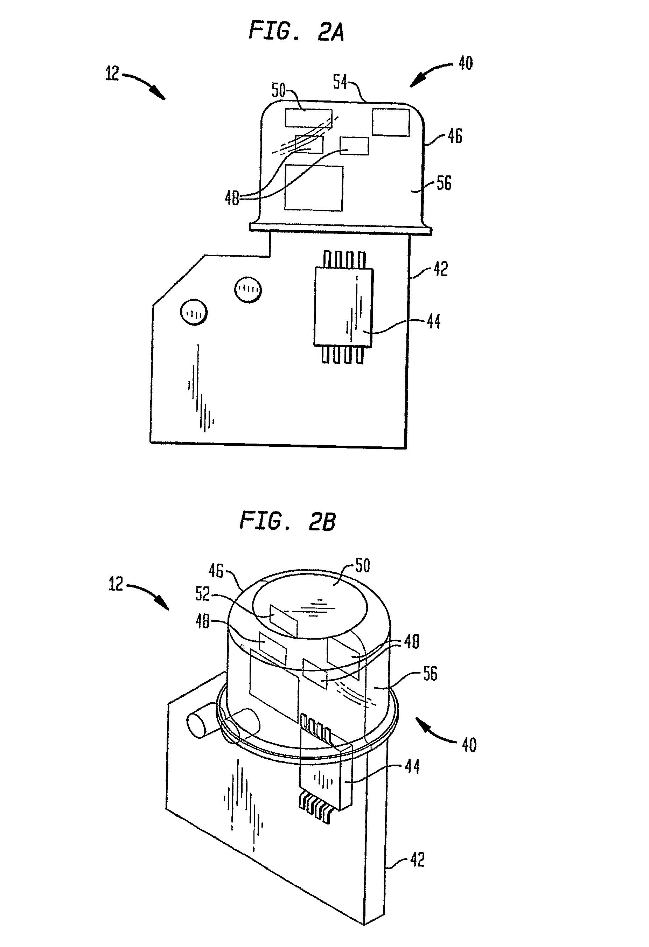

Treatment device 10 of the illustrated embodiment includes body 14 and replaceable treatment tip 12. Replaceable treatment tip 12 is used to transfer the heat energy from treatment device 10 to the patient's skin. Replaceable treatment tip 12 will be described in greater detail with reference to Figures 2a and 2b, but generally includes heat transfer, or treatment surface 16 and tip housing 18.

Replaceable treatment tip 12 connects to body 14 using tip engagement mechanism 20, which engages and holds treatment tip 12 in the proper orientation with respect to body 14. End 38 of treatment tip 12 is configured to selectively engage with body 14 to ensure the proper orientation of treatment tip 12. Tip engagement mechanism 20 includes electrical connectors 22 which provide electrical connection between the electrical components in treatment tip 12 and the electrical components in body 14.

Body 14 includes power button 24 and treatment button 26. Power button 14 is used to turn treatment device 10 on and off Treatment button 26 is used to initiate and/or cancel treatments. Treatment button 26 can include light emitting diodes (LEDs) 28 that indicate whether treatment device 10 is ready to begin a treatment. While the illustrated embodiment is shown using LEDs as a display, any display technology such as LCDs or other display may be used without departing from the concepts described herein. For example, LEDs 28 could include an amber light to indicate that the device is not ready to begin a treatment and a green light to indicate that treatment device 10 is ready to begin a treatment. LEDs 32 provide additional visual information to the user, such as the charge remaining in the battery, the number of remaining treatments available using the current replaceable treatment tip 12, the type of treatment tip 12 currently connected to body 14, and any other information which may be useful or interesting to the user. A speaker, not shown, is also housed in body 14. The speaker can be used to provide audible information to the user such as the amount of time remaining in the treatment, an error condition, low battery charge, and any other audible information that might be useful or interesting to the user.

Housing 30 of body 14 provides a protective cover to hold the internal components of treatment device 10.

Housing 30 holds the internal electrical components and the power source, such as rechargeable batteries. While treatment device 10 is described as using rechargeable batteries as the preferred power source, any suitable power source may be used by embodiments of the invention, including receiving power from an ordinary wall socket using a power cord. Battery charge port 34 is used to plug in a charger to charge the internal batteries. A data port 36 can be included in body 14. Data port 36 allows treatment device 10 to communicate with another device, such as a computer or PDA, and allows the internal electrical components to receive new programs or new data to be used in treatment device 10.

Referring now to Figures 2a and 2b, replaceable treatment tip 12 is shown with tip housing 18 from Figure 1 removed. Replaceable treatment tip 12 of the illustrated embodiment includes thermal mass 40 which is used to transfer the heat energy of the applied treatment. Thermal mass 40 includes thermal transfer cup 46. Thermal transfer cup 46 has treatment surface 54 which is placed in contact with the region of skin that is being treated. Thermal transfer cup 46 is preferably formed from hard anodized aluminum, but could be formed from any material with suitable heat transfer properties. Thermal transfer cup 46 encloses a tip of circuit board 42.

Circuit board 42 has electrical components used to perform the treatment mounted on its surface and provides the electrical contact between treatment tip 12 and body 14 of treatment device 10 shown in Figure 1. Resistors 48 are mounted onto the tip of circuit board 42 enclosed by thermal transfer cup 46 and are used to convert electrical energy from the batteries to heat energy used in the treatment. Resistors 48 are brought into thermal communication with thermal treatment cup 46 by means of a thermally conductive potting compound 56 that at least partially fills thermal transfer cup 46 and holds thermal transfer cup 46 onto circuit board 42. Potting compound 56 of the illustrated embodiment provides the majority of thermal mass for thermal mass 40. Thermal mass 40 is preferably be chosen to balance competing factors that come into play during each treatment.

Thermal mass 40 is preferably be chosen large enough so that the treatment device can reach and hold as closely as possible to a constant treatment temperature during the entire treatment cycle. If the thermal mass is too small, the treatment device is unable to maintain a constant temperature when treatment surface 50 is brought into contact with the patient's skin which acts a heat sink, drawing heat out of thermal mass 40 as resistors 48 are unable to generate enough heat quickly enough to replace the heat transferred to the patient's skin. This also increases the chances of overshoot, where the temperature of the thermal mass, in trying to replace the heat transferred to the treatment site, overshoots the intended treatment temperature causing the thermal mass to reach a temperature higher than the intended temperature. As the treatment temperature is set very close to the temperature that would burn skin, overshoot can easily result in burns to the patient and must be avoided. Too small a thermal mass, therefore, would require a lower set temperature to increase the safety margin used to ensure that the thermal mass does not overshoot to a dangerous temperature, and a lower set temperature may not be as effective in treating the targeted skin condition.

Conversely, if thermal mass 40 is chosen too large, it becomes hard to control as the thermal mass becomes slow to respond to input changes. Additionally, if the thermal mass is too large, the amount of power needed to bring it to the operating temperature becomes larger, either reducing the number of treatments that can be performed by a single battery charge or requiring a larger battery capacity. Thermal mass 40, therefore, should be chosen to provide sufficient thermal mass to allow a well regulated treatment to occur at a set temperature.

Control of the temperature of thermal mass 40 is done in response to signals from thermister 50, mounted on circuit board 42 in potting compound 56. Thermister 50 provides an electrical signal indicative of the temperature of thermal mass 40 to a microprocessor in body 14 of Figure 1. Thermister 50 is mounted as close as possible to treatment surface 54 such that the signal provided by thermister 50 most closely indicates the temperature of thermal mass 40 at the treatment site. Mounting thermistor 50 further from treatment surface 54 might allow treatment surface 54 to exceed the treatment temperature despite readings from the thermister due to thermal gradients within thermal mass 40.

In addition to thermister 50, redundant thermister 52 is provided in thermal mass 40 of embodiments to ensure that a failure of thermister 50 cannot result in thermal mass 40 reaching temperatures that could be dangerous to the patient. Redundant thermister 52 can be connected to the same microprocessor as thermister 50, but is preferably connected to a separate safety circuit outside of the control loop of thermister 50. This provides another level of safety to the treatment device as redundant thermister 52 would not be affected by a failure in the main control loop that includes thermister 50 and the microprocessor.

Treatment tip 12 also includes memory element 44 mounted to circuit board 42. Memory element 44 can be any combination of processing and memory elements utilized to store and implement treatment tip specific functions. Memory element 44 is used to store tip specific information. One example of such tip specific information would be a limit to the number of uses for each replaceable treatment tip. Treatment tips can lose their ability to strictly maintain the treatment temperatures as the elements within the treatment tip are altered through repeated heating and cooling cycles. As a result, treatment tips are only intended for a limited number of treatments. Memory element 44 is able to track the number of treatments for a treatment tip and to disable a treatment tip after the predetermined number of treatment cycles.

Additionally, memory element 44 of the illustrated embodiment includes calibration information for its associated treatment tip. As the individual components used in each treatment tip have their own variances from their marked values, each tip is calibrated during manufacturing to provide calibration information stored in memory element 44 and used to adjust the heating algorithm of treatment device 10 to account for the particular values of the components in the treatment tip.

The memory element can also store treatment variables such as treatment cycle duration, treatment temperature and treatment frequency based on the particular lesion the tip is designed to treat. In addition to the cycle information, calibration information and treatment variables, other treatment tip specific information can be stored in memory element 44 that aids the treatment device in its operation. Such information can, for example, be information that identifies the type of tip and the intended treatment protocols, as well as algorithm information used during a treatment cycle.

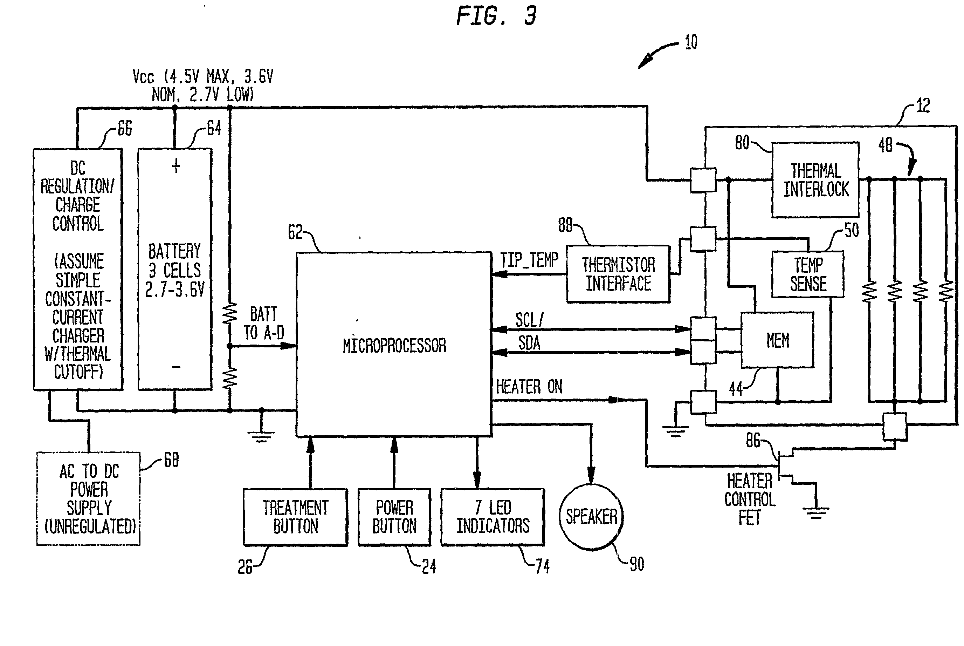

Referring now to Figure 3, a diagram showing the operation of the electrical components of the treatment device 10 from Figure 1 is described. Device 10 includes microprocessor 62. Microprocessor 62 is programmed to respond to and control the various inputs and outputs of treatment device 10 from Figure 1. Microprocessor 62 receives input from power button 24, and in response operates to power-up or power-down the treatment device accordingly. Microprocessor 62 also receives input from treatment button 70 and operates to start or stop treatment based on input from treatment button 70. LEDs 74 are turned on and off by microprocessor 62 to communicate visual information to information to the user, while speaker 90 is controlled by microprocessor 62 to communicate audible information to the user.

Microprocessor 62 is also connected to treatment tip 12. Microprocessor communicates with memory element 44 and exchanges information on tip cycles, calibration, treatment variations and other tip specific information. Microprocessor also receives the signal from thermister 50 through interface 88. Using the signal from thermister 50, microprocessor 62 is operable to control the temperature of the thermal mass of treatment tip 12. Microprocessor 62 of the illustrated embodiment is connected to the gate of field effect transistor ("FET") 86, and by varying the voltage at the gate of FET 86 is able to control the amount of current flowing through resistors 48. The heat produced by resistors 48 is proportional to the amount of current passing through them. Thermal interlock 80 provides a safety mechanism to ensure that the failure of thermister 50 does not cause a dangerous operating temperature in the treatment tip.

Microprocessor 62 is programmed with a control algorithm referred to as a proportional, integral, derivative or PID. A PID is a control algorithm which uses three modes of operation: the proportional action is used to dampen the system response, the integral corrects for droop, and the derivative prevents overshoot and undershoot. The PID algorithm implemented in Microprocessor 62 operates to bring the thermal mass to the desired operating temperature as quickly as possible with minimal overshoot, and also operates to respond to changes in the temperature of the thermal mass during the treatment cycle that are caused by the heat sink effect of the treatment area.

In addition to being connected to FET 86, resistors 48 are connected to battery 64 through thermal interlock 80, which can be a fuse having a maximum current rating chosen to prevent runaway overheating of resistors 48. Battery 64, which can be comprised of one or more individual cells, is charged by battery charger 66 when battery charger 66 is connected to external power supply 68. External power supply 68 can be any type of power supply, but is normally an AC to DC converter connected between battery charger 66 and an ordinary wall outlet. According to embodiments, the output voltage of battery 66 is directly related to the amount of charge left in battery 66, therefore, by monitoring the voltage across battery 66 microprocessor 62 can determine the amount of charge remaining in battery 66 and convey this information to the user using LEDs 74 or speaker 90. Other methods of determining battery voltages or charge for different battery technologies can also be used and are well within the scope of the present invention.

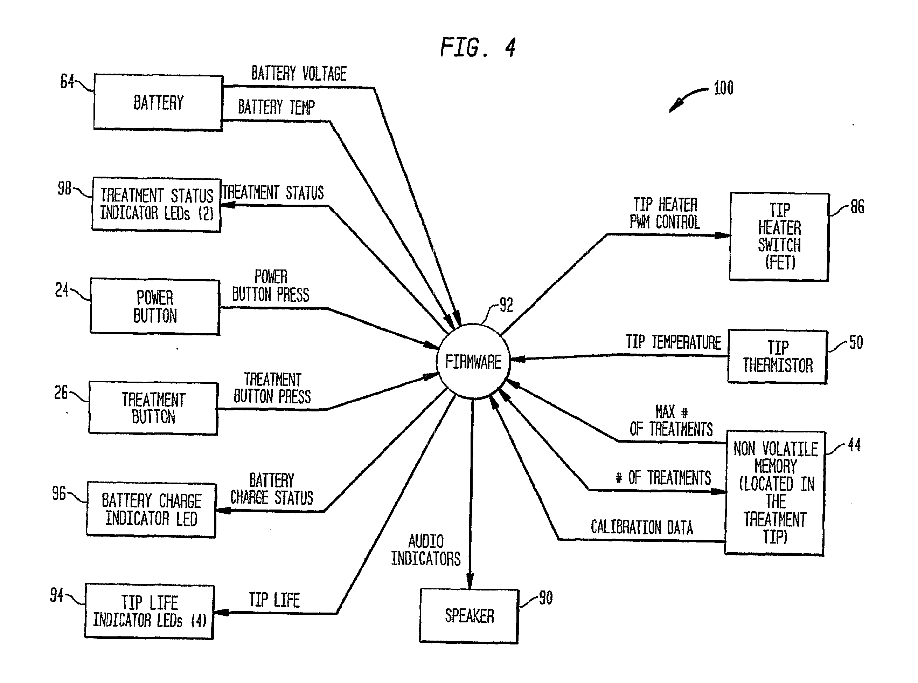

Referring now to Figure 4, a diagram showing the various inputs to the Firmware run by microprocessor 62 of Figure 3 is described. Firmware 92 represents the programming loaded on microprocessor 62 from Figure 3. As described with reference to Figure 3, microprocessor 62 is operable to respond to and control the various aspects of treatment device 10 from Figure 1. Firmware 92 is able to accept inputs from power button 70, treatment button 26, tip thermister 50 and battery 64. Firmware 92 is also able to exchange information with memory element 44, such as the maximum number of treatments for the tip, the number of tip treatments actually performed by the tip, and calibration data. The microprocessor 62 and memory element 44 may exchange any other information that may increase the efficacy of treatment device 10.

In response to the thermister input and information from memory element 44, firmware 92 controls FET 86 to regulate the temperature of the thermal mass in the treatment tip according to the PID algorithm programmed into firmware 92. Firmware 92 also controls speaker 90 to provide audible feedback to the user and LEDs 94, 96, and 98 which are subsets of LEDs 74 from Figure 3, and provide indications of tip life (LEDs 94), battery charge (LED 96), and treatment status (LEDs 98).

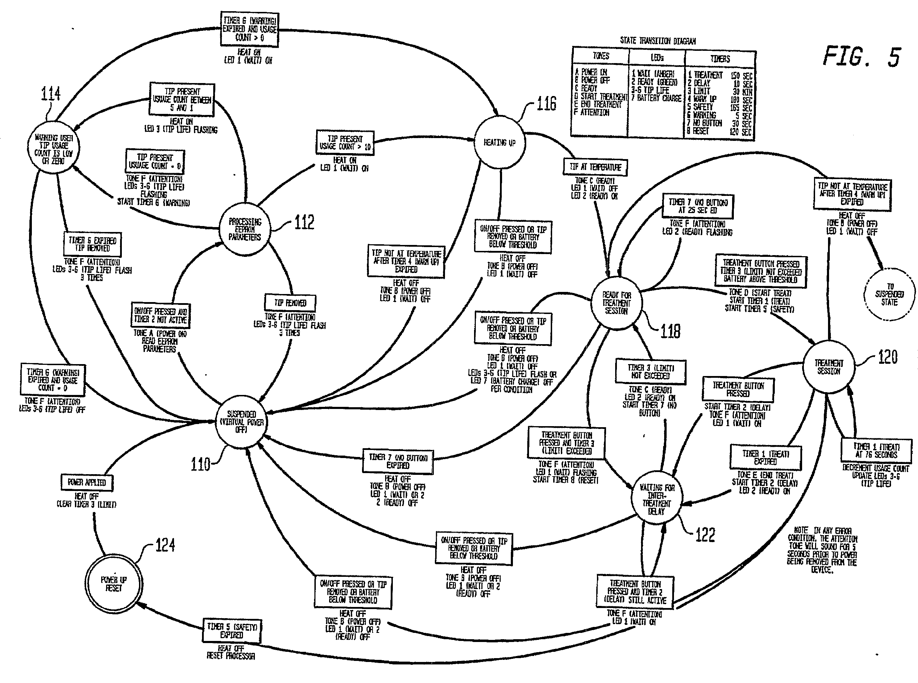

Referring now to Figure 5 a state transition diagram showing various operating states of firmware 92 from Figure 4 according to an embodiment is described. The state diagram begins a Suspended state 110 which is the power off state. During the power off mode the microprocessor is still receiving some power to allow it to monitor the power button. The Suspended state 110 is left when the power on button is pressed, and the state proceeds to the Processing Tip Memory state 112. In the Processing Tip Memory state 112 the microprocessor 62 and memory element 44 from Figure 3 exchange tip specific treatment information. If the tip usage count is not low or zero, the state passes to Heating state 116. If the tip count is found to be low or zero the state progresses to the Warning state which provides visual and or audible signals to the user to indicate that the tip count is low or zero. If the tip count is zero or the tip is removed, the state passes from the Warning state 114 to the Suspended state 110. If the tip count is low, but not zero the state passes from the Warning state 114 to the Heating state 116.

During the Heating state 116 the tip is heated using resistors 48 from Figure 3. A predictive model is used to set a timer based on the amount of time that should be required for the tip to come to temperature. This timer acts as in indicator that the thermal mass is responding to the heating correctly. If the tip does not reach the predetermined operating temperature by the expiration of the timer, it is an indication of a potentially faulty component and the treatment device shuts down by transitioning to Suspended state 110. Other predictions of thermal mass behavior can also be used to detect potentially faulty components.

In addition to the expiration of the timer, the treatment device powers down by transitioning to the Suspended state if the power button is pressed, the tip is removed or the battery voltage falls below a threshold, and indication of the fault is provided to the user through visual and/or audible signals. If the tip successfully reaches the operating temperature within the designated time the state transitions to Ready state 118. A timer is started upon entering the Ready state 118. If the timer expires or the power button is pressed while in the Ready state 118, the state transitions to the Suspended state 110.

If the treatment button is pressed while in Ready state 118 the state transitions to Treatment state 120. Two timers, a treatment timer and a safety timer, are started upon entering the Treatment state 120. The safety timer is slightly longer than the treatment timer so that if there is a failure in the treatment timer the safety timer will expire and transition the state to the Power Reset state 124 before transitioning to the Suspended state 110. The state also transitions from Treatment state 120 to Suspended state 110 if the power button is pressed during a treatment cycle.

As a treatment cycle can be a relatively long period of time, the treatment device can also be programmed to provide visual and/or audible indications of the progress of the treatment timer. For example, speaker 90 of Figure 3 can be used to provide intermittent tones during the treatment to let the user know that the treatment is continuing. The time between the tones could be spaced to provide an indication of the remaining time in the treatment cycle, such as by shortening the time between the tones as the cycle gets closer to the end. Many other methods of providing visual or audible feedback could be contemplated and are well within the scope of the present invention.

When the treatment timer expires, or if the treatment button is pressed, the state transitions from Treatment state 120 to Wait state 122 which forces an inter-treatment delay. If the power button is pressed or the tip removed during the Wait state, the state transitions to Suspended state 110. After the expiration of the inter-treatment delay the state transitions back to Ready state 118. In addition to the inter-treatment delay, the Wait state 122 can be used to force a temporal treatment limit. While the inter-treatment delay forces a relatively brief delay between treatment cycles, the temporal treatment limit acts to limit the number of treatments that can be performed in specified period. For example, if the treatment cycle is two and a half minutes and the inter-treatment delay is 10 seconds, a temporal treatment limit of 30 minutes could be used to limit the device to approximately 10 to 11 consecutive treatments before a forced interval is imposed.

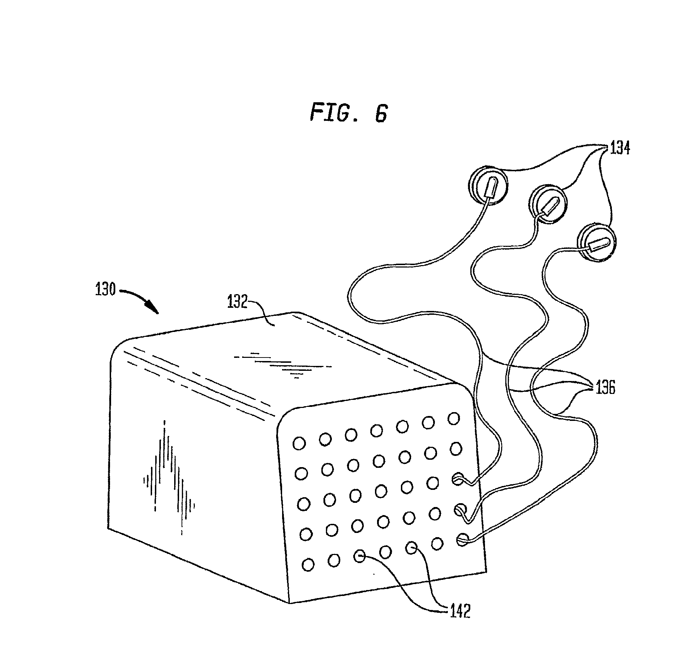

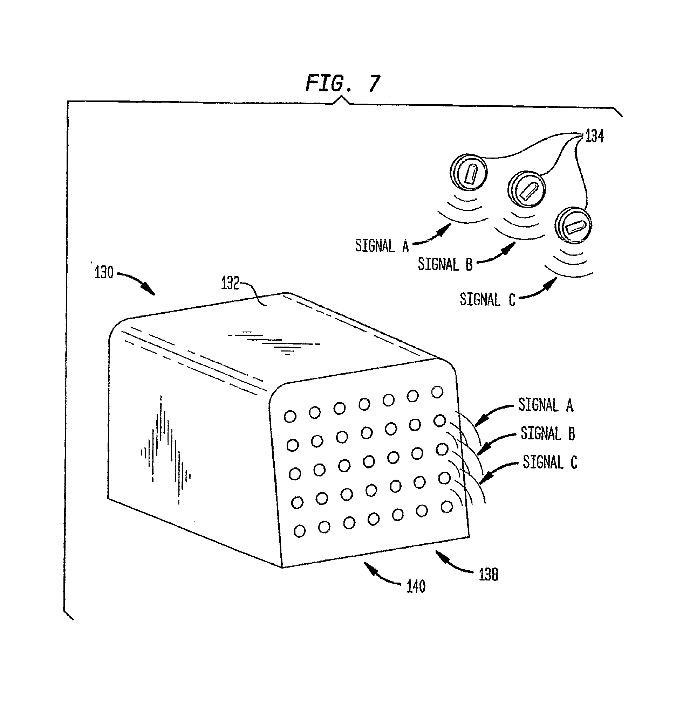

Referring now to Figures 6 and 7, a treatment device capable of treating multiple locations simultaneously is described. Multi-location treatment device 130 includes treatment base station 132 and multiple treatment dots 134. Base station 132 includes all the essential functionality of the body 14 of treatment device 10 from Figure 1, while treatment dots 134 each contain the essential functionality of replaceable treatment tip 12 from Figure 1. The change between treatment device 10 of Figure 1 and multi-location treatment device 10 is primarily a change in form factor to accommodate multiple treatment locations simultaneously.

Base station 132 communicates with treatment dots 134 either by wires 136 connecting the base station 132 to the treatment dots 134, as is shown in Figure 6, or by means of a wireless connection as is shown in Figure 7 using wireless transmitters 142. The base station 132 also includes LEDs used to convey visual information to the user of device 130. Base station 132 and treatment dots operate in the same manner as described above for the single tip treatment system. Treatment dots 134 can be formed with an adhesive coating on the treatment surface to allow treatment dots 134 to adhere to the treatment area.

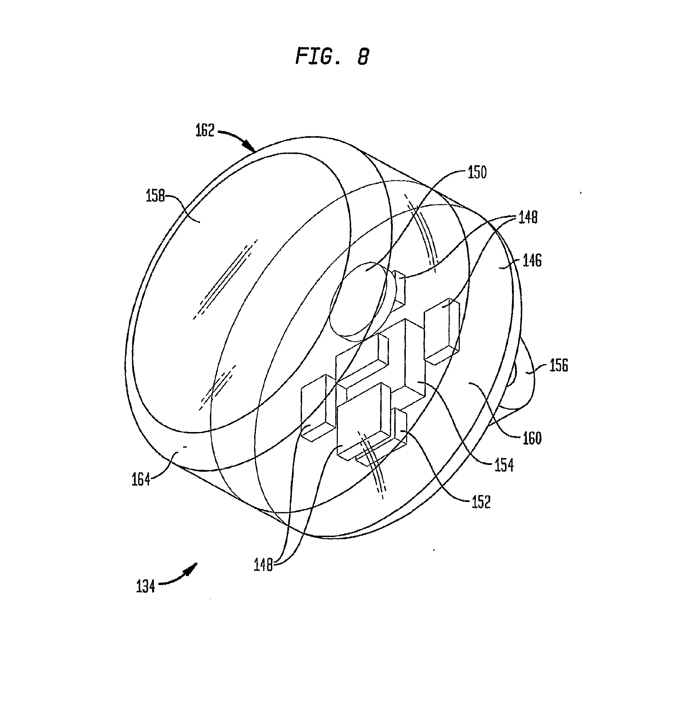

Referring now to Figure 8, a treatment dot such as one of treatment dots 134 from Figures 6 and 7 according to embodiments is shown in greater detail. Treatment dot 134 includes housing 146, which holds the electrical components mounted on circuit board 160. As with tip 12 from Figure 2, the circuit board and electrical components are covered with a thermally conductive potting compound to create a thermal mass. Mounted on circuit board 160 are memory and logic element 154, resistors 148 and thermister 152, which operate as similarly as described with respect to Figure 2. In addition, treatment dot 134 includes port 156 which can either be the connection point for wire 136 from Figure 6, or can hold a wireless transceiver for use with the wireless treatment device shown in Figure 7. In the wireless device power source 150 might also be utilized to supply the power necessary for resistors 148 to generate the desired heat.

Treatment dot 134 shown in Figure 8 also illustrates an alternative treatment surface. Treatment surface 54 from Figure 2 was described as formed from aluminum or other rigid thermally conductive material. A rigid surface such as aluminum works very well when used against a deformable surface such as skin. However, against a non-deformable surface, such as a fingernail or toenail, the rigid treatment surface is unable to contact all of the surface to be treated. As a result heat is not transferred to the entire treatment area. To over come this limitation, a deformable treatment surface and thermal mass can be used such that the treatment surface and thermal mass conform to the treatment area. One embodiment of such a deformable treatment tip is illustrated by tip 162. Tip 162 is formed by a deformable material 158 such as silicon or similar material formed by a thermally conductive material 164 which is also deformable, such as a silicon or similar gel. Resistors 148 are able to transfer heat to the thermal material 164 and deformable surface 158.

Other tip designs could easily be contemplated and are well within the scope of the present invention. Other tip designs could include treatment surfaces in the form of pads with larger surface areas to treat larger regions of skin. While the treatment surface of the present invention can be applied directly to the skin of a subject, there can also be one or more intervening layers between the skin of the subject and the treatment surface. The intervening layer or layers can be any desired substance capable of allowing transmission of thermal energy. For example, an intervening layer can be composed of a solid, semi-solid or liquid layer such as the gel described above, or such an intervening layer can be a sterilizable or disposable covering for the treatment surface which is intended to prevent the transmission of infectious agent from one use to the next.

The treatment device can be sterilized by heating the device to a high enough temperature and for a sufficient period of time to result in loss of viability of any microorganisms that are present on the treatment surface. The treatment surface itself can be sterilized by conventional methods of sterilization such as application of an antiseptic to the surface to be sterilized.

The shape of the treatment surface can be any shape and composed of any material that is appropriate for the treatment of a particular type of lesion. In particular, where the interface is composed of a inflexible or substantially inflexible material, the interface can be substantially planar, convex or concave. For example, a treatment surface intended for the treatment of pustular eruptions or localized abscesses on the face might preferably be substanially planar or convex so as to come in contact with one or more lesions and possibly their immediate surrounding. A treatment surface for the treatment of fungal infections of the toenail might preferably be shaped as a ring, arc, cap, or other appropriate shape so as to be placed in close proximity to the infected tissue. Other shapes for the treatment surface will be readily apparent, depending on the types of lesions intended to be treated with a treatment device in accordance with the present invention.

The concepts described herein also contemplate a calibration method for calibrating the treatment tip. In prior calibration systems, power would be applied to the tip resistors to heat the tip, and the result would be measured with an outside temperature sensor, such as an infrared thermometer or a contact thermister. The resulting temperature reading would be compared with what the an expected thermister value. In a preferred embodiment of the calibration method according to the concepts described herein, the tip is buried un-powered in a pre-heated thermal mass, such as a heated aluminum block with receiving holes milled into it, that is being held at a constant temperature. The tip's thermister value is determined and used to calculate a calibration constant.

Thermal Aspect Ratio

The aspect ratio between a thermal transfer area and thermal contact area plays a significant role in determining the internal skin temperature resulting from a give size of thermal treatment tip. This Thermal Aspect Ratio should be used to design appropriate treatment devices, as well as to drive predictive models on given design specifications.

The treatment device of the present invention relies on a thermal contact tip used to heat a limited region of the skin to a temperature sufficient to induce heat shock in bacterial, viral or fungal lesions. The size and temperature of the tip are tuned to result in a carefully targeted temperature which is sufficient to induce heat shock, but not high enough to create significant or permanent skin damage.

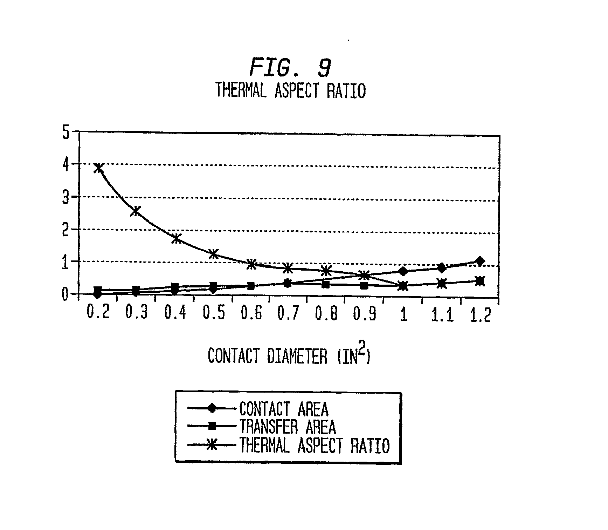

The existing research on heat transfer and contact burns focuses on a fixed (and relatively large) contact area (typically 7 cm$^2$ or larger). These research studies attempt to create predictive models of burn incidence at varying temperatures and times. Reducing the size of the contact area, however, can produce a dramatic reduction in burn incidence. This reduction does not occur in a linear relationship. This non-linear relationslip is largely the result of the Thermal Aspect Ratio shown in Figure 9, which is also non-linear as a result of the inherent geometry of the two components (contact area and transfer area). As the diameter of the contact area increases, the ratio of the transfer area to the contact area drops off dramatically at first and then much more gradually as the contact diameter goes above 0.60 inches.

Since the contact area increases with the square of the contact radius and the transfer area is essentially a fixed width band around the circumference of the contact area, it follows that drop in the Thermal Aspect Ratio is initially steep.

This analysis relies on a fixed heat transfer coefficient for skin. The presumption is that this fixed coefficient results in a fixed transfer area width (0.125") which is the area immediately surrounding the circumference of the contact area through which the higher temperature of the contact area is wicked away and dissipated through contact with the air and blood-circulating skin mass. Because of the fixed heat transfer coefficient, the transfer area acts much like a fence, preventing additional heat transfer beyond that which is permitted by its own heat transfer coefficient.

When the Thermal Aspect Ratio is high, the contact area gets relatively good and uniform heat dissipation via the transfer area (in addition to the heat transfer via blood flow and body mass contact directly beneath the contact area). As the Thermal Aspect Ratio drops, a larger and larger contact area takes on more and more heat energy which cannot be dissipated via the transfer area resulting in rapid heat buildup. In essence, larger contact areas lose their ability to shed heat and ramp up to higher temperatures at a rapidly increasing rate.

The Thermal Aspect Ratio dynamic, therefore, creates an inflection point in contact tip design. Before the inflection point (tip diameters below a certain point), a relatively high capacity for dissipation allows the use of higher temperature therapy to a concentrated area without significant risk of thermal damage. Beyond the inflection point (tip diameters above a certain point) maintaining a safe and predictable temperature becomes more and more difficult to do and tip operating temperature (and therefore, therapy temperature) must come down in order to avoid thermal damage.

Preferred Set Temperature and Treatment Time

To determined the preferred set temperature and treatment time, two factors are preferably considered. First, the set temperature and treatment time must be sufficient to cause thermal damage to the infectious agent or irritant causing the skin lesion being treated. Second, the set temperature and treatment time must be below the threshold that would damage the skin being treated. The first factor is discussed with reference to Examples 1-3 below using exemplary infectious agents. Based on Examples 1-3 a set temperature of 121°F (49.44°C) for a period of 150 seconds proves to be effective for a variety of infectious agent and irritants. While a set temperature of 121°F and a treatment time of 150 seconds are chosen for an embodiment of the present invention, other embodiments using combinations of set temperatures and treatment times which depart significantly from the described embodiment are well within the scope of the present invention.

To ensure that the described embodiment of a set time and temperature do not cause burn damage to the treatment area modeling can be performed against previous research done into burn injuries. The modeling assumes that the skin surface in contact with the applicator immediately reaches the applicator temperature of 121 °F and remains at that temperature for the entire 150 seconds. First, the set temperature and treatment time are plotted against the Time-Surface Temperature Thresholds plot represented in Figure 4, page 711 from Moritz and Henriques, "Studies of Thermal Energy," American Journal of Pathology, 1947, Vol. 23, pp. 695-720. The plot of 49.44 °C at 150 seconds is just under the dashed curve representing "the first morphological evidence of thermal damage," such as slight reddening. At the set temperature, the curve indicates that the first reversible damage occurs at 195 seconds. Thus, according to Moritz and Herniques, the set temperature and treatment time are safe, and at worse might produce slight reddening of the treatment area.

Based on the data of Moritz and Henriques cited above, Xu and Qian in an article entitled "Analysis of Thermal Injury Process Based on Enzyme Deactivation Mechanisms," in Journal of Biomechanical Engineering, Transactions of the ASME, Vol. 117, pp. 462-465 (1995) developed an equation for a damage function, $\Omega$, based on enzyme deactivation concepts.

$$\Omega = \int_{o}^{t} \frac{1*10^{-4}\exp(100z)}{1+8*10^{4}\exp(-195z)}dt$$

where z = 1-305.65/T °K, and t is in seconds
In this model T=322.59 °K and is constant, therefore, $\Omega=4.947*10^{-3}*\Delta t=0.742$ for 150 seconds.

Example 1

Temperature dependent death curves for P. acnes.

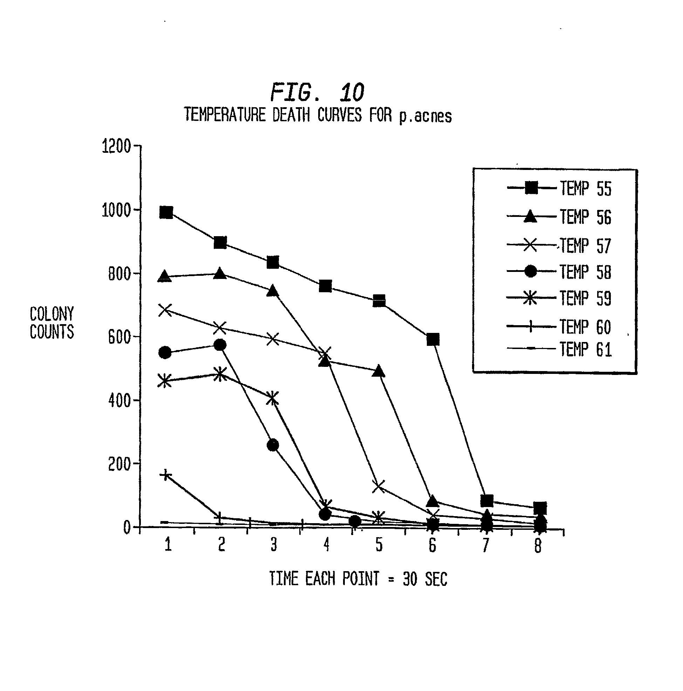

Materials and Methods: The bacterial strain P. acnes was purchased from The American Type Culture Collection ATCC (No. 11827, Lot 419571, Manassas, VA). The cultures were stored in KWIK-STIK lyophilized preparations. The lyophilized cells (P.acnes) were rehydrated according to the manufacturers recommendations and initially grown on a streak plate to isolate individual colonizes under anaerobic conditions. These plates were then incubated overnight at 37° C in an anaerobic chamber. Individual colonies were then isolated and inoculated into TSB-growth media with medium agitation overnight. From these aliquots of 0.1 ml of TSB broth culture was added to the 0.9 ml of PBS sterile buffer. This mixture was then transferred to thin-walled Eppendorf 1.5 ml tubes and placed in a heating block at various times and temperatures. The cultures after specific incubation times were removed and 0.1 ml of the material was plated onto TSA plates. This mixture was then spread with a sterile hockey-stick and then allowed to incubate at 37° for 5 days in anaerobic conditions. The plates were then removed and colonies were counted and recorded. The results are demonstrated in Figure 10. Figure 10 demonstrates the rapid decline of P. acnes in response to various temperatures and duration of treatment. The baseline P. acnes colony count that had not been exposed to the heat source was 1050.

Results: A general trend of reduction of required time to kill the bacterial strain is seen at higher temperature incubations. Also of note is the temporal thermal threshold where the number of colonies drops off in a very steep fashion. By using the curves generated by such experiments the optimal thermal output and the timing for each temperature can be extrapolated for a localized heating device. The in vitro data shown demonstrates significant sensitivity of P. acnes bacterial cells to the effects of sustained low-level heat. Temperatures of 55°C result in the death of substantially all of the bacteria after 3½ minutes. Temperatures of 58 and 59°C result in the death of substantially all of the bacteria after 2 minutes. These curves demonstrate that P. acnes can be rendered largely non-viable by treatment under the conditions shown by the death curves.

Example 2

Treatment of acne lesions in human subjects. The inventors have performed preliminary studies on over 100 volunteers experiencing outbreaks of acne lesions. All subjects reported being satisfied with the results obtained. The results showed a clear response to treatment in approximately 90% of subjects treated. No subject reported any serious adverse effects due to treatment. Furthermore, we have discovered that a treated lesion heals more than 80% faster than untreated lesions.

The electrical device used in the present study had an interface of approximately 0.4 cm2. The interface of the device was heated to a constant temperature of approximately 48-50°C prior to application of the device to the skin surface, and the temperature was maintained during the treatment period. Each of the subjects were given instructions on how to use the device and were monitored during the treatment. The treatment consisted of a 2½ minute application of the device to the lesion site. The study called for the application of two treatment cycles to each patient, with the second treatment cycle being administered 12 hours after the first. In practice, however, the treatments were frequently only conducted once on each subject because twelve hours after the first treatment many of the lesions had healed to an extent that they did not require any further treatment.

Results of experiments performed on volunteer subjects are listed in Table 1. Members of the control group were not treated. Members of the treatment group were treated as described above. Both groups either examined or self-reported the results of treatment over the following 14 days. Only results from study participants who reported data for 14 days were included in the table. The data is reported in terms of the size of the lesion prior to treatment. A lesion size of 100% indicates that the lesion size was unchanged. Lesion size was approximated in increments of 10%. A lesion size of 0% indicates that the lesion had fully healed.

EP 2 389 885 A1

| Control Group | | | | | | | | | | | | | | | | | | |
|---|---|---|---|---|---|---|---|---|---|---|---|---|---|---|---|---|---|---|
| | | | | | Day | Day | Day | Day | Day | Day | Day | Day | Day | Day | Day | Day | Day | Day |
| # | Name | Gender | Age | | 1 | 2 | 3 | 4 | 5 | 6 | 7 | 8 | 9 | 10 | 11 | 12 | 13 | 14 |
| 1 | LEF | F | 27 | | 100% | 100% | 100% | 100% | 90% | 90% | 80% | 80% | 50% | 20% | 10% | 0% | 0% | 0% |
| 2 | AMC | F | 22 | | 100% | 100% | 100% | 90% | 90% | 80% | 80% | 60% | 40% | 40% | 20% | 20% | 20% | 10% |
| 3 | AWC | F | 16 | | 100% | 100% | 100% | 100% | 100% | 100% | 100% | 80% | 80% | 60% | 40% | 10% | 10% | 10% |
| 4 | KAC | F | 13 | | 100% | 100% | 100% | 80% | 80% | 70% | 40% | 40% | 40% | 40% | 20% | 10% | 0% | 0% |
| 5 | ECP | F | 35 | | 100% | 100% | 100% | 100% | 80% | 80% | 80% | 20% | 20% | 20% | 20% | 10% | 0% | 0% |
| 6 | KSL | F | 21 | | 100% | 100% | 90% | 90% | 80% | 80% | 60% | 60% | 60% | 30% | 30% | 10% | 10% | 0% |
| 7 | NET | F | 18 | | 100% | 100% | 100% | 80% | 80% | 80% | 60% | 60% | 60% | 30% | 30% | 30% | 10% | 10% |
| 8 | LHJ | F | 27 | | 100% | 100% | 100% | 80% | 80% | 80% | 50% | 50% | 50% | 50% | 20% | 10% | 10% | 0% |
| 9 | TAA | F | 28 | | 100% | 90% | 90% | 90% | 90% | 70% | 70% | 70% | 40% | 30% | 30% | 10% | 10% | 10% |
| | | | Total | | 100% | 99% | 98% | 90% | 86% | 81% | 69% | 58% | 49% | 36% | 24% | 12% | 8% | 4% |
| | | | | | | | | | | | | | | | | | | |
| 1 | ZAC | M | 15 | | 100% | 100% | 100% | 100% | 80% | 80% | 60% | 60% | 60% | 40% | 30% | 30% | 10% | 0% |
| 2 | ZMP | M | 14 | | 100% | 100% | 100% | 100% | 90% | 90% | 90% | 80% | 80% | 60% | 60% | 20% | 20% | 10% |
| 3 | MAP | M | 18 | | 100% | 100% | 100% | 100% | 90% | 90% | 90% | 70% | 70% | 70% | 30% | 30% | 10% | 0% |
| 4 | CDC | M | 40 | | 100% | 100% | 90% | 80% | 70% | 70% | 30% | 30% | 30% | 10% | 10% | 0% | 0% | 0% |

| | Control Group | | | | | | | | | | | | | | | | | |
|---|---|---|---|---|---|---|---|---|---|---|---|---|---|---|---|---|---|---|
| | | | | | Day | Day | Day | Day | Day | Day | Day | Day | Day | Day | Day | Day | Day | Day |
| # | Name | Gender | Age | | 1 | 2 | 3 | 4 | 5 | 6 | 7 | 8 | 9 | 10 | 11 | 12 | 13 | 14 |
| 5 | CAC | M | 24 | | 100% | 100% | 100% | 90% | 80% | 80% | 80% | 50% | 50% | 50% | 20% | 20% | 10% | 0% |
| 6 | RAA | M | 33 | | 100% | 100% | 100% | 90% | 80% | 70% | 70% | 60% | 60% | 40% | 20% | 20% | 10% | 10% |
| | | | Total | | 100% | 100% | 98% | 93% | 82% | 80% | 70% | 58% | 58% | 45% | 28% | 20% | 10% | 3% |
| | | | | | | | | | | | | | | | | | | |
| | | | Totals | | 100% | 99% | 98% | 91% | 84% | 81% | 69% | 58% | 53% | 39% | 26% | 15% | 9% | 4% |

| | | | | | | | | | | | | | | | | | | |
|---|---|---|---|---|---|---|---|---|---|---|---|---|---|---|---|---|---|---|
| | | | | | **Treatment Group** | | | | | | | | | | | | | |
| | | | | | Day | Day | Day | Day | Day | Day | Day | Day | Day | Day | Day | Day | Day | Day |
| **#** | **Name** | **Gender** | **Age** | | **1** | **2** | **3** | **4** | **5** | **6** | **7** | **8** | **9** | **10** | **11** | **12** | **13** | **14** |
| 1 | AAS | F | 34 | | 100% | 30% | 20% | 10% | 0% | 0% | 0% | 0% | 0% | 0% | 0% | 0% | 0% | 0% |
| 2 | ACC | F | 36 | | 100% | 20% | 0% | 0% | 0% | 0% | 0% | 0% | 0% | 0% | 0% | 0% | 0% | 0% |
| 3 | AWC | F | 40 | | 100% | 70% | 30% | 10% | 0% | 0% | 0% | 0% | 0% | 0% | 0% | 0% | 0% | 0% |
| 4 | BAB | F | 27 | | 100% | 10% | 0% | 0% | 0% | 0% | 0% | 0% | 0% | 0% | 0% | 0% | 0% | 0% |
| 5 | CAB | F | 29 | | 100% | 0% | 0% | 0% | 0% | 0% | 0% | 0% | 0% | 0% | 0% | 0% | 0% | 0% |
| 6 | CHH | F | 30 | | 100% | 60% | 60% | 40% | 10% | 0% | 0% | 0% | 0% | 0% | 0% | 0% | 0% | 0% |
| 7 | DSF | F | 33 | | 100% | 0% | 0% | 0% | 0% | 0% | 0% | 0% | 0% | 0% | 0% | 0% | 0% | 0% |
| 8 | GDL | F | 34 | | 100% | 40% | 10% | 0% | 0% | 0% | 0% | 0% | 0% | 0% | 0% | 0% | 0% | 0% |
| 9 | HCD | F | 14 | | 100% | 50% | 20% | 0% | 0% | 0% | 0% | 0% | 0% | 0% | 0% | 0% | 0% | 0% |
| 10 | HLL | F | 36 | | 100% | 0% | 0% | 0% | 0% | 0% | 0% | 0% | 0% | 0% | 0% | 0% | 0% | 0% |
| 11 | JLP | F | 19 | | 100% | 20%. | 0% | 0% | 0% | 0% | 0% | 0% | 0% | 0% | 0% | 0% | 0% | 0% |
| 12 | JSH | F | 28 | | 100% | 20% | 20% | 0% | 0% | 0% | 0% | 0% | 0% | 0% | 0% | 0% | 0% | 0% |
| 13 | JUL | F | 31 | | 100% | 70% | 50% | 30% | 10% | 0% | 0% | 0% | 0% | 0% | 0% | 0% | 0% | 0% |
| 14 | KAC | F | 13 | | 100% | 50% | 30% | 10% | 0% | 0% | 0% | 0% | 0% | 0% | 0% | 0% | 0% | 0% |
| 15 | KDJ | F | 20 | | 100% | 20% | 0% | 0% | 0% | 0% | 0% | 0% | 0% | 0% | 0% | 0% | 0% | 0% |
| 16 | KEF | F | 26 | | 100% | 10% | 0% | 0% | 0% | 0% | 0% | 0% | 0% | 0% | 0% | 0% | 0% | 0% |
| 17 | KFC | F | 17 | | 100% | 0% | 0% | 0% | 0% | 0% | 0% | 0% | 0% | 0% | 0% | 0% | 0% | 0% |
| 18 | KST | F | 33 | | 100% | 80% | 80% | 60% | 30% | 10% | 0% | 0% | 0% | 0% | 0% | 0% | 0% | 0% |
| 19 | LEF | F | 21 | | 100% | 30% | 10% | 10% | 0% | 0% | 0% | 0% | 0% | 0% | 0% | 0% | 0% | 0% |
| 20 | LKD | F | 34 | | 100% | 50% | 50% | 50% | 30% | 30% | 20% | 10% | 10% | 0% | 0% | 0% | 0% | 0% |
| 21 | LKJ | F | 15 | | 100% | 70% | 40% | 20% | 10% | 0% | 0% | 0% | 0% | 0% | 0% | 0% | 0% | 0% |
| 22 | MDD | F | 35 | | 100% | 20% | 0% | 0% | 0% | 0% | 0% | 0% | 0% | 0% | 0% | 0% | 0% | 0% |
| 23 | MDF | F | 19 | | 100% | 50% | 10% | 0% | 0% | 0% | 0% | 0% | 0% | 0% | 0% | 0% | 0% | 0% |

(continued)

| | | | | | Day 1 | Day 2 | Day 3 | Day 4 | Day 5 | Day 6 | Day 7 | Day 8 | Day 9 | Day 10 | Day 11 | Day 12 | Day 13 | Day 14 |
|---|---|---|---|---|---|---|---|---|---|---|---|---|---|---|---|---|---|---|
| **#** | **Name** | **Gender** | **Age** | | | | | | | | | | | | | | | |
| 24 | MEA | F | 38 | | 100% | 70% | 30% | 20% | 20% | 10% | 0% | 0% | 0% | 0% | 0% | 0% | 0% | 0% |
| 25 | MLJ | F | 29 | | 100% | 60% | 30% | 10% | 0% | 0% | 0% | 0% | 0% | 0% | 0% | 0% | 0% | 0% |
| 26 | NJM | F | 37 | | 100% | 50% | 40% | 10% | 0% | 0% | 0% | 0% | 0% | 0% | 0% | 0% | 0% | 0% |
| 27 | RTY | F | 23 | | 100% | 10% | 0% | 0% | 0% | 0% | 0% | 0% | 0% | 0% | 0% | 0% | 0% | 0% |
| 28 | SAH | F | 18 | | 100% | 40% | 10% | 0% | 0% | 0% | 0% | 0% | 0% | 0% | 0% | 0% | 0% | 0% |
| 29 | SAL | F | 14 | | 100% | 50% | 10% | 0% | 0% | 0% | 0% | 0% | 0% | 0% | 0% | 0% | 0% | 0% |
| 30 | SBH | F | 18 | | 100% | 20% | 20% | 10% | 0% | 0% | 0% | 0% | 0% | 0% | 0% | 0% | 0% | 0% |
| 31 | SFH | F | 35 | | 100% | 0% | 0% | 0% | 0% | 0% | 0% | 0% | 0% | 0% | 0% | 0% | 0% | 0% |
| 32 | SLB | F | 31 | | 100% | 60% | 30% | 30% | 10% | 100% | 0% | 0% | 0% | 0% | 0% | 0% | 0% | 0% |
| 33 | TCA | F | 16 | | 100% | 0% | 0% | 0% | 0% | 0% | 0% | 0% | 0% | 0% | 0% | 0% | 0% | 0% |
| 34 | TDB | F | 25 | | 100% | 20% | 0% | 0% | 0% | 0% | 0% | 0% | 0% | 0% | 0% | 0% | 0% | 0% |
| 35 | TEM | F | 38 | | 100% | 60% | 30% | 30% | 10% | 10% | 10% | 0% | 0% | 0% | 0% | 0% | 0% | 0% |
| 36 | TLS | F | 13 | | 100% | 80% | 40% | 20% | 10% | 10% | 10% | 0% | 0% | 0% | 0% | 0% | 0% | 0% |
| 37 | TSJ | F | 36 | | 100% | 50% | 30% | 10% | 10% | 0% | 0% | 0% | 0% | 0% | 0% | 0% | 0% | 0% |
| 38 | VYM | F | 21 | | 100% | 80% | 80% | 80% | 50% | 30% | 10% | 10% | 10% | 0% | 0% | 0% | 0% | 0% |
| | | | Total | | 100% | 37% | 21% | 12% | 5% | 5% | 1% | 1% | 1% | 0% | 0% | 0% | 0% | 0% |
| | | | | | | | | | | | | | | | | | | |
| 1 | CAC | M | 40 | | 100% | 20% | 10% | 0% | 0% | 0% | 0% | 0% | 0% | 0% | 0% | 0% | 0% | 0% |
| 2 | CDM | M | 39 | | 100% | 60% | 40% | 10% | 10% | 0% | 0% | 0% | 0% | 0% | 0% | 0% | 0% | 0% |
| 3 | DAD | M | 16 | | 100% | 20% | 10% | 0% | 0% | 0% | 0% | 0% | 0% | 0% | 0% | 0% | 0% | 0% |
| 4 | DDL | M | 21 | | 100% | 0% | 0% | 0% | 0% | 0% | 0% | 0% | 0% | 0% | 0% | 0% | 0% | 0% |
| 5 | DFB | M | 35 | | 100% | 80% | 80% | 40% | 20% | 10% | 10% | 10% | 10% | 0% | 0% | 0% | 0% | 0% |
| 6 | EHE | M | 14 | | 100% | 20% | 0% | 0% | 0% | 0% | 0% | 0% | 0% | 0% | 0% | 0% | 0% | 0% |

EP 2 389 885 A1

| | | | | | Day 1 | Day 2 | Day 3 | Day 4 | Day 5 | Day 6 | Day 7 | Day 8 | Day 9 | Day 10 | Day 11 | Day 12 | Day 13 | Day 14 |
|---|---|---|---|---|---|---|---|---|---|---|---|---|---|---|---|---|---|---|---|
| | | | | | | | | | | | | | | | | | | | |
| # | Name | Gender | Age | | | | | | | | | | | | | | | | |
| 7 | HAF | M | 33 | | 100% | 60% | 60% | 20% | 20% | 10% | 10% | 0% | 0% | 0% | 0% | 0% | 0% | 0% |
| 8 | JEY | M | 15 | | 100% | 20% | 20% | 10% | 0% | 0% | 0% | 0% | 0% | 0% | 0% | 0% | 0% | 0% |
| 9 | JKG | M | 18 | | 100% | 40% | 10% | 10% | 0% | 0% | 0% | 0% | 0% | 0% | 0% | 0% | 0% | 0% |
| 10 | KEG | M | 36 | | 100% | 0% | 0% | 0% | 0% | 0% | 0% | 0% | 0% | 0% | 0% | 0% | 0% | 0% |
| 11 | KSP | M | 31 | | 100% | 30% | 30% | 10% | 10% | 0% | 0% | 0% | 0% | 0% | 0% | 0% | 0% | 0% |
| 12 | MJP | M | 34 | | 100% | 20% | 20% | 10% | 0% | 0% | 0% | 0% | 0% | 0% | 0% | 0% | 0% | 0% |
| 13 | OAP | M | 20 | | 100% | 90% | 40% | 20% | 10% | 0% | 0% | 0% | 0% | 0% | 0% | 0% | 0% | 0% |
| 14 | PLT | M | 38 | | 100% | 70% | 50% | 30% | 10% | 10% | 0% | 0% | 0% | 0% | 0% | 0% | 0% | 0% |
| 15 | RAA | M | 21 | | 100% | 20% | 20% | 0% | 0% | 0% | 0% | 0% | 0% | 0% | 0% | 0% | 0% | 0% |
| 16 | RDC | M | 30 | | 100% | 30% | 10% | 10% | 0% | 0% | 0% | 0% | 0% | 0% | 0% | 0% | 0% | 0% |
| 17 | RCJ | M | 25 | | 100% | 60% | 20% | 20% | 20% | 10% | 0% | 0% | 0% | 0% | 0% | 0% | 0% | 0% |
| 18 | TFL | M | 16 | | 100% | 0% | 0% | 0% | 0% | 0% | 0% | 0% | 0% | 0% | 0% | 0% | 0% | 0% |
| 19 | SHT | M | 28 | | 100% | 20% | 10% | 0% | 0% | 0% | 0% | 0% | 0% | 0% | 0% | 0% | 0% | 0% |
| 20 | DKP | M | 36 | | 100% | 50% | 10% | 10% | 0% | 0% | 0% | 0% | 0% | 0% | 0% | 0% | 0% | 0% |
| 21 | WRT | M | 28 | | 100% | 30% | 10% | 0% | 0% | 0% | 0% | 0% | 0% | 0% | 0% | 0% | 0% | 0% |
| 22 | WJK | M | 32 | | 100% | 80% | 80% | 60% | 40% | 40% | 20% | 20% | 10% | 10% | 0% | 0% | 0% | 0% |
| 23 | PLL | M | 24 | | 100% | 20% | 0% | 0% | 0% | 0% | 0% | 0% | 0% | 0% | 0% | 0% | 0% | 0% |
| 24 | MWT | M | 31 | | 100% | 0% | 0% | 0% | 0% | 0% | 0% | 0% | 0% | 0% | 0% | 0% | 0% | 0% |
| 25 | TTM | M | 26 | | 100% | 10% | 10% | 0% | 0% | 0% | 0% | 0% | 0% | 0% | 0% | 0% | 0% | 0% |
| 26 | BTL | M | 37 | | 100% | 60% | 30% | 10% | 10% | 0% | 0% | 0% | 0% | 0% | 0% | 0% | 0% | 0% |
| 27 | DWD | M | 22 | | 100% | 70% | 20% | 20% | 10% | 0% | 0% | 0% | 0% | 0% | 0% | 0% | 0% | 0% |

Treatment Group

| Treatment Group | | | | | | | | | | | | | | | | | | |
|---|---|---|---|---|---|---|---|---|---|---|---|---|---|---|---|---|---|---|
| | | | | | Day | Day | Day | Day | Day | Day | Day | Day | Day | Day | Day | Day | Day | Day |
| # | Name | Gender | Age | | 1 | 2 | 3 | 4 | 5 | 6 | 7 | 8 | 9 | 10 | 11 | 12 | 13 | 14 |
| | | | Total | | 100% | 36% | 22% | 11% | 6% | 3% | 1% | 1% | 1% | 0% | 0% | 0% | 0% | 0% |
| | | | | | | | | | | | | | | | | | | |
| | | | Totals | | 100% | 37% | 21% | 11% | 6% | 4% | 1% | 1% | 1% | 0% | 0% | 0% | 0% | 0% |

Example 3

The inventors have tested the device on multiple oral herpes lesions of human volunteers, and the results have shown a complete termination of the herpetic lesion after two applications of the device at 2 1/2 minutes per treatment, 12 hours apart, as described in Example 2. The volunteers reported a marked decrease in healing time after treatment versus the usual healing cycle for lesions of this type.

All of the compositions and methods disclosed and claimed herein can be made and executed without undue experimentation in light of the present disclosure. While the compositions and methods of this invention have been described in terms of preferred embodiments, it will be apparent to those of skill in the art that variations can be applied to the devices or methods and in the steps or in the sequence of steps of the methods described herein without departing from the concept, spirit and scope of the invention. More specifically, it will be apparent that certain mechanical elements related to those described above can be substituted for the mechanical elements described herein to achieve the same or similar results. All such similar substitutes and modifications apparent to those skilled in the art are deemed to be within the spirit, scope and concept of the invention as defined by the appended claim.

Although the present invention and its advantages have been described in detail, it should be understood that various changes, substitutions and alterations can be made herein without departing from the spirit and scope of the invention as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, compositions of matter, means, methods, or steps, presently existing or later to be developed that perform substantially the same function or achieve substantially the same result as the corresponding embodiments described herein may be utilized according to the present invention. Accordingly, the appended claims are intended to include within their scope such processes, machines, manufacture, compositions of matter, means, methods, or steps.

The following is a non-exhaustive list of embodiments.

(1) A treatment device for treating localized skin conditions on a patient, the treatment device comprising:

a thermally conductive surface designed to be placed in contact with the skin of the patient;
a temperature sensor adjacent to the thermally conductive surface;
a heating element operable to heat the thermally conductive surface; and
a controller electrically connected to the heating element and the temperature sensor, the controller operable to control the heating element in response to a signal from the temperature sensor.

(2) The treatment device of embodiment (1) wherein the thermally conductive surface is interchangeable or disposable.

(3) The treatment device of embodiment (1) wherein the controller is capable of maintaining the interface at a temperature between about 48°C to about 53°C for at least 30 seconds.

(4) The treatment device of embodiment (1) wherein the controller is capable of maintaining the interface at a temperature between about 48°C to about 53°C for at least 60 seconds.

(5) The treatment device of embodiment (1) wherein the controller is capable of maintaining the interface at a temperature between about 48°C to about 53°C for at least 90 seconds.

(6) The treatment device of embodiment (1) wherein the controller is capable of maintaining the interface at a temperature between about 48°C to about 53°C for at least 120 seconds.

(7) The treatment device of embodiment (1) wherein the controller is capable of maintaining the interface at a temperature between about 48°C to about 53°C for at least 150 seconds.

(8) The treatment device of embodiment (1) wherein the controller uses a feedback control mechanism involving the use of PID control algorithms.

(9) The treatment device of embodiment (1) wherein the controller is capable of controllably ramping the temperature of the thermally conductive surface to the desired temperature range.

(10) A treatment device comprising:

a replaceable treatment tip including a thermal mass and a heating element in thermal contact with the thermal mass, the thermal mass having a contact surface; and
a body connectable to the replaceable treatment tip, the body including a controller operable to control the heating element to heat the contact surface of the thermal mass to an operating temperature.

(11) The treatment device of embodiment (10) wherein the replaceable treatment tip includes a temperature sensor to provide a signal to the controller indicative of the temperature of the contact surface.

(12) The treatment device of embodiment (10) wherein the replaceable treatment tip includes a memory storing calibration information specific to the replaceable treatment tip.

(13) The treatment device of embodiment (10) wherein the replaceable treatment tip includes a memory storing a number of treatment cycles for which the replaceable treatment tip has been used.

(14) The treatment device of embodiment (13) wherein the controller monitors the number of treatment cycles for a particular replaceable treatment tip by reading the memory in the particular replaceable treatment tip and wherein the controller prevents the use of the particular replaceable treatment tip when the number of treatment cycles exceeds a predetermined number.

(15) The treatment device of embodiment (14) wherein the controller provides a signal indicating when the number of treatment cycles for a particular replaceable treatment tip approaches the predetermined number.

(16) The treatment device of embodiment (10) wherein the replaceable treatment tip includes a thermal interlock to prevent overheating of the contact surface.

(17) The treatment device of embodiment (10) wherein the operating temperature is a temperature between about 48°C to about 53°C.

(18) The treatment device of embodiment (10) wherein the controller includes a plurality of pre-programmed settings, each pre-programmed setting intended to treat a different condition.

(19) The treatment device of embodiment (10) further comprising a plurality of indicators operable to provide an indication of the status of the treatment device.

(20) The treatment device of embodiment (19) wherein the plurality of indicators includes both visual and audible indicators.

(21) The treatment device of embodiment (10) further comprising a rechargeable batter, wherein the rechargeable batter provides power to the controller and the heating element.

(22) A replaceable tip for a treatment device, the replaceable tip comprising:

a thermally conductive contact surface;
a heating element in thermal contact with the contact surface;
a temperature sensor in thermal contact with the contact surface;
a memory element storing treatment tip specific information related to the replaceable tip, the information including a maximum number of treatment cycles for the replaceable tip; and
a connector operable to connect the heating element, temperature sensor, and memory element to a controller.

(23) The replaceable tip of embodiment (22) wherein the temperature sensor is a thermister.

(24) The replaceable tip of embodiment (22) wherein the memory elenient stores calibration information specific to the replaceable treatment tip.

(25) The replaceable tip of embodiment (22) wherein the memory element stores a number corresponding to the number of treatment cycles for which the replaceable treatment tip has been used.

(26) The replaceable tip of embodiment (22) wherein the replaceable treatment tip includes a thermal interlock to prevent overheating of the contact surface.

(27) The replaceable tip of embodiment (22) further comprising a thermal mass in thermal contact with the heating element and contact surface.

(28) The replaceable tip of claim (27) wherein the thermal mass comprising thermal potting compound.

(29) The replaceable tip of claim (28) wherein at least the heating element and temperature sensor are encased in the thermal potting compound.

(30) A method for determining an optimum configuration for a treatment device, the treatment device for treating skin lesions by applying a heated contact surface having a surface area, at a treatment temperature for a predetermined treatment time, the method comprising:

selecting values for two variables selected from a group of three variables, the
group of three variables comprising: the treatment temperature, the treatment time, and the surface area; and
determining an optimum value for a third variable fiom the group of three variables.

(31) The method of embodiment (30) wherein the surface area of the heated contact surface is chosen according to its thermal aspect ratio.

(32) The method of embodiment (31) wherein the thermal aspect ration is determined fiom the ratio between a contact area for the heated contact surface and a thermal transfer area of the heated surface.

(33) A method for determining a fault condition in replaceable treatment tip, the replaceable treatment tip for treating skin conditions through application of heat generated by a heating element in the replaceable treatment tip, the method comprising:

predicting a model behavior for the treatment tip in response applied heat from the heating element;
monitoring an actual behavior of the replaceable treatment tip in response to the applied heat; and
determining if the treatment tip is faulty based on a comparison of the model behaviour and the actual behaviour.

(34) The method of embodiment (33) wherein the model behavior is stored in a memory device in the replaceable treatment tip.

(35) The method of embodiment (33) wherein the replaceable treatment tip is connectable to a controller during operation, the controller operable to determine if the treatment tip is faulty.

(36) A method for calibration of a replaceable treatment tip comprising:

heating the replacement treatment tip using an external thermal mass to a known set point;
measuring a resulting resistance value for a thermister in the replacement treatment tip;
determining the difference between the resulting resistance value and an expected resistance value for the therrnister; and
determining a correction factor for the therrnister based on the difference.

**Claims**

1. A treatment device comprising:

   a replaceable treatment tip including a thermal mass and a heating element in thermal contact with the thermal mass, the thermal mass having a contact surface; and
   a body connectable to the replaceable treatment tip, the body including a controller operable to control the heating element to heat the contact surface of the thermal mass to an operating temperature.

2. The treatment device of claim 1 wherein the replaceable treatment tip includes a temperature sensor to provide a signal to the controller indicative of the temperature of the contact surface.

3. The treatment device of claim 1 wherein the replaceable treatment tip includes a memory storing calibration information specific to the replaceable treatment tip.

4. The treatment device of claim 1 wherein the replaceable treatment tip includes a memory storing a number of treatment cycles for which the replaceable treatment tip has been used.

5. The treatment device of claim 4 wherein the controller monitors the number of treatment cycles for a particular replaceable treatment tip by reading the memory in the particular replaceable treatment tip and wherein the controller prevents the use of the particular replaceable treatment tip when the number of treatment cycles exceeds a predetermined number.

6. The treatment device of claim 5 wherein the controller provides a signal indicating when the number of treatment cycles for a particular replaceable treatment tip approaches the predetermined number.

7. The treatment device of claim 1 wherein the replaceable treatment tip includes a thermal interlock to prevent overheating of the contact surface.

8. The treatment device of claim 1 wherein the operating temperature is a temperature between about 48°C to about 53°C.

9. The treatment device of claim 1 wherein the controller includes a plurality of pre-programmed settings, each pre-programmed setting intended to treat a different condition.

10. The treatment device of claim 1 further comprising a plurality of indicators operable to provide an indication of the status of the treatment device.

11. The treatment device of claim 10 wherein the plurality of indicators includes both visual and audible indicators.

12. The treatment device of claim 1 further comprising a rechargeable battery, wherein the rechargeable battery provides power to the controller and the heating element.

13. A replaceable tip for a treatment device, the replaceable tip comprising:

a thermally conductive contact surface;
a heating element in thermal contact with the contact surface;
a temperature sensor in thermal contact with the contact surface;
a memory element storing treatment tip specific information related to the replaceable tip, the information including a maximum number of treatment cycles for the replaceable tip; and
a connector operable to connect the heating element, temperature sensor, and memory element to a controller.

14. The replaceable tip of claim 13 wherein the temperature sensor is a thermister.

15. The replaceable tip of claim 13 wherein the memory element stores calibration information specific to the replaceable treatment tip.

16. The replaceable tip of claim 13 wherein the memory element stores a number corresponding to the number of treatment cycles for which the replaceable treatment tip has been used.

17. The replaceable tip of claim 13 wherein the replaceable treatment tip includes a thermal interlock to prevent overheating of the contact surface.

18. The replaceable tip of claim 13 further comprising a thermal mass in thermal contact with the heating element and contact surface.

19. The replaceable tip of claim 18 wherein the thermal mass comprising thermal potting compound.

**20.** The replaceable tip of claim 19 wherein at least the heating element and temperature sensor are encased in the thermal potting compound.

## FIG. 1

## FIG. 2A

## FIG. 2B

FIG. 3

EP 2 389 885 A1

# FIG. 4

64 — BATTERY

BATTERY VOLTAGE

BATTERY TEMP

98 — TREATMENT STATUS INDICATOR LEDs (2)

TREATMENT STATUS

24 — POWER BUTTON

POWER BUTTON PRESS

26 — TREATMENT BUTTON

TREATMENT BUTTON PRESS

96 — BATTERY CHARGE INDICATOR LED

BATTERY CHARGE STATUS

94 — TIP LIFE INDICATOR LEDs (4)

TIP LIFE

AUDIO INDICATORS

SPEAKER — 90

FIRMWARE — 92

100

TIP HEATER PWM CONTROL

TIP HEATER SWITCH (FET) — 86

TIP TEMPERATURE

TIP THERMISTOR — 50

MAX # OF TREATMENTS

# OF TREATMENTS

CALIBRATION DATA

NON VOLATILE MEMORY (LOCATED IN THE TREATMENT TIP) — 44

EP 2 389 885 A1

FIG. 5

FIG. 6

## FIG. 7

FIG. 8

FIG. 9
THERMAL ASPECT RATIO

CONTACT DIAMETER (IN$^2$)

- ◆ CONTACT AREA
- ■ TRANSFER AREA
- ✻ THERMAL ASPECT RATIO

*FIG. 10*
TEMPERATURE DEATH CURVES FOR p.acnes

Europäisches
Patentamt

European
Patent Office

Office européen
des brevets

# EUROPEAN SEARCH REPORT

Application Number

EP 11 17 4303

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/030332 A1 (KNOWLTON EDWARD W [US] ET AL) 12 February 2004 (2004-02-12) | 1-6, 8-16, 18-20 | INV.<br>A61B18/04<br>A61F7/00 |
| Y | * abstract; figures 1A-2 *<br>* paragraphs [0021] - [0028] *<br>* paragraphs [0046], [0068] - [0101] * | 7,17 | |
| X | US 6 350 262 B1 (ASHLEY JOHN E [US])<br>26 February 2002 (2002-02-26)<br>* the whole document * | 1,2 | |
| Y | US 6 162 217 A (KANNENBERG DONALD P [US] ET AL) 19 December 2000 (2000-12-19)<br>* abstract; figures 1-3 *<br>* columns 3, 6 * | 7,17 | |
| A | US 4 090 517 A (TAKENAKA NAGATOKI)<br>23 May 1978 (1978-05-23)<br>* abstract; claims 1-7; figures 1-2D *<br>* column 2 * | 1-20 | |
| | | | **TECHNICAL FIELDS SEARCHED (IPC)**<br><br>A61B<br>A61F |

The present search report has been drawn up for all claims

| Place of search | Date of completion of the search | Examiner |
|---|---|---|
| Munich | 24 October 2011 | Scheffler, Arnaud |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    ...............................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 11 17 4303

This annex lists the patent family members relating to the patent documents cited in the above-mentioned European search report.
The members are as contained in the European Patent Office EDP file on
The European Patent Office is in no way liable for these particulars which are merely given for the purpose of information.

24-10-2011

| Patent document cited in search report | | Publication date | Patent family member(s) | | Publication date |
|---|---|---|---|---|---|
| US 2004030332 | A1 | 12-02-2004 | AU | 2003302939 A1 | 21-10-2004 |
| | | | BR | PI0403032 A | 28-06-2005 |
| | | | CA | 2474891 A1 | 30-09-2004 |
| | | | CN | 1697631 A | 16-11-2005 |
| | | | EP | 1558164 A2 | 03-08-2005 |
| | | | JP | 2006521889 A | 28-09-2006 |
| | | | KR | 20050118247 A | 16-12-2005 |
| | | | US | 2004030332 A1 | 12-02-2004 |
| | | | US | 2006206110 A1 | 14-09-2006 |
| | | | WO | 2004090939 A2 | 21-10-2004 |
| US 6350262 | B1 | 26-02-2002 | AU | 1086599 A | 10-05-1999 |
| | | | US | 6176857 B1 | 23-01-2001 |
| | | | US | 6350262 B1 | 26-02-2002 |
| | | | WO | 9920191 A1 | 29-04-1999 |
| US 6162217 | A | 19-12-2000 | AU | 4205800 A | 02-11-2000 |
| | | | US | 6162217 A | 19-12-2000 |
| | | | US | 2001029369 A1 | 11-10-2001 |
| | | | WO | 0062695 A1 | 26-10-2000 |
| US 4090517 | A | 23-05-1978 | JP | 53006088 U | 19-01-1978 |
| | | | US | 4090517 A | 23-05-1978 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6183500 B

### Non-patent literature cited in the description

- **YONKOSKY, D.M ; P.E. POCHI.** Acne vulgaris in childhood. Pathogenesis and management. *Dermatol Clin,* 1986, vol. 4 (1), 127-36
- **CUNLIFFE, W.J. et al.** Comedogenesis: some aetiological, clinical and therapeutic strategies. *Dermatology,* 2003, vol. 206 (1), 11-6
- **SCHOLDGEN, W.** *Hautarzt,* 1965, vol. 16 (11), 518-20
- **LEVER, L. ; R. MARKS.** *Drugs,* 1990, vol. 3 9 (5), 681-92
- **LEVER, L. ; R. MARKS.** *Drugs,* 1990, vol. 39 (5), 681-92
- **RUSSELL, J.J.** *Am Fam Physician,* 2000, vol. 61 (2), 357-66
- **GOLLNICK, H.P. ; A. KRAUTHEIM.** *Dermatology,* 2003, vol. 206 (1), 29-36
- **LOVECKOVA, Y. ; I. HAVLIKOVA.** *Biomed Pap Med Fac Univ Palacky Olomouc Czech Repub,* 2002, vol. 146 (2), 29-32
- **VERMEULEN, B. ; J.P. REMON ; H. NELIS.** *Int J Pharm,* 1999, vol. 178 (1), 137-41
- **RIZER, R.L. et al.** Clindamycin phosphate 1% gel in acne vulgaris. *Adv Ther,* 2001, vol. 18 (6), 244-52
- **THORNE, E.G.** *Br J Dermatol,* 1992, vol. 127 (41), 31-6
- **SCHOLDGEN, W.** *Z Allgemeinmed,* 1972, vol. 48 (17), 833-5
- **MELSKI, J.W. ; K.A. ARNDT.** Current concepts: topical therapy for acne. *N Engl J Med,* 1980, vol. 302 (9), 503-6
- **LESTER, R.S.** Topical formulary for the pediatrician. *Pediatr Clin North Am,* 1983, vol. 30 (4), 749-65
- **BRONIARCZYK-DYLA, G. ; C. ARKUSZEWSKA.** *Dermatol Monatsschr,* 1989, vol. 175 (1), 40-3
- **ZANDER, E. ; S. WEISMAN.** Treatment of acne vulgaris with salicylic acid pads. *Clin Ther,* 1992, vol. 14 (2), 247-53
- **KAYE, E.T. ; K.M. KAYE.** Topical antibacterial agents. *Infect Dis Clin North Am,* 1995, vol. 9 (3), 547-59
- **RUIZ-ESPARZA, J. ; J.B. GOMEZ.** *Dermatol Surg,* 2003, vol. 29 (4), 333-9
- **PEPALL, L.M. ; M.P. COSGROVE ; W.J. CUNLIFFE.** *Br J Dermatol,* 1991, vol. 125 (3), 256-9
- **MORITZ ; HENRIQUES.** Studies of Thermal Energy. *American Journal of Pathology,* 1947, vol. 23, 695-720
- Analysis of Thermal Injury Process Based on Enzyme Deactivation Mechanisms. *Journal of Biomechanical Engineering, Transactions of the ASME,* 1995, vol. 117, 462-465